(19) Europäisches Patentamt
European Patent Office
Office européen des brevets

(11) **EP 3 266 371 A1**

(12) **EUROPEAN PATENT APPLICATION**

(43) Date of publication:
**10.01.2018 Bulletin 2018/02**

(51) Int Cl.:
***A61B 5/01*** *(2006.01)*

(21) Application number: **16178313.9**

(22) Date of filing: **07.07.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Cowmatix S.r.l.**
**20123 Milano (IT)**

(72) Inventors:
- **MIODINI, Marzio**
  **20010 CORNAREDO (MI) (IT)**
- **SALA, Leonardo**
  **20010 CORNAREDO (MI) (IT)**

(74) Representative: **Robba, Pierpaolo**
**Interpatent S.R.L.**
**Via Caboto, 35**
**10129 Torino (IT)**

# (54) METHOD OF AND APPARATUS FOR DIAGNOSING LEG PATHOLOGIES IN QUADRUPEDS

(57) An automatic method of diagnosing pathologies of the distal parts of the limbs of a quadruped is based on the processing (101 - 109) of thermographic images of such limbs. The processing includes the following steps: identifying (103, 104), in each thermographic image and for each limb concerned by the diagnosis, an area containing the distal part, and extracting an identified image of the distal part from said area; validating (104) identified images complying with predetermined criteria as images utilisable for diagnostic purposes; extracting (105) features that are significant for the detection of the presence and the kind of pathology from the validated images; and classifying (106) the distal part of a limb as unaffected by pathologies or as affected by a specific pathology on the basis of such features. There are also provided an apparatus implementing the method and an information technology product containing program codes for implementing the method when the product is loaded into a processing device.

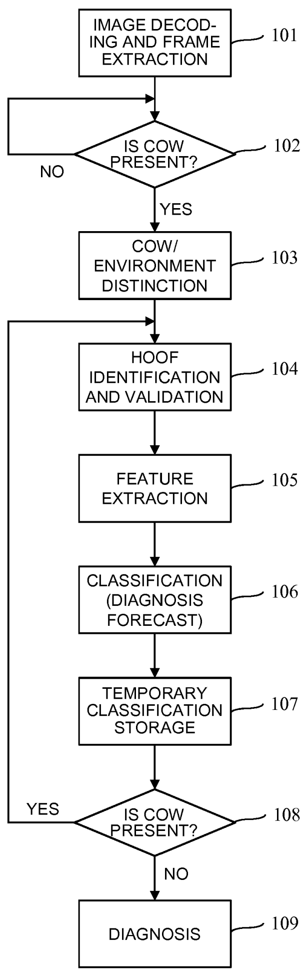


FIG. 6

EP 3 266 371 A1

## Description

*Technical Field*

The invention concerns a method of and an apparatus for diagnosing limb pathologies in quadrupeds, especially, but not exclusively, dairy cows. More particularly, the invention relates to a method and an apparatus based upon the thermographic analysis of the surfaces of the distal parts of the legs or limbs, especially, but not exclusively, the rear limbs.

*Prior Art*

It is known that pathologies affecting the distal parts of cattle limbs, in particular the hoofs, are a source of economic losses, due both to the additional costs associated with veterinary expenses and to a reduction in the value of the animal, for instance because of the decrease in milk production capacity, as well as to the reduction in life expectation and the need of an early replacement of the animal. An early diagnosis of such pathologies is therefore essential in order to minimise the risk of such economic losses.

Systems proposed in recent years for diagnosing pathological conditions in the limbs, in particular the hoofs, of quadrupeds are based on the thermographic analysis of the concerned area and exploit the fact that lesions or other pathological conditions cause a local increase of the surface temperature in the concerned area.

Thermography is a non-invasive diagnostic tool that measures infrared radiation emitted by an object and is arranged to display the information in image form. Colour variations on the image are related to different temperatures. Conventionally, hotter regions are shown in red or white, whereas cooler regions are shown in blue or black. Thermography is based on the use of an instrument capable of generating a plurality of signals representative of the intensity of the electromagnetic radiation emitted by a body in the infrared range. Such an instrument is known as thermal camera or infrared camera. It is an instrument allowing generating infrared photographic images. Typically, a thermal camera is capable of operating with electromagnetic emissions having wavelengths in the range 3 to 15 $\mu$m. The thermal camera exploits the well-known Planck's law according to which the temperature of an emitting body is proportional to the radiation emitted. The thermographic image obtained by means of a thermal camera is representative of the temperature distribution over the surface of an object. The thermal camera measures the infrared radiation received within its visual field (for instance 7 to 15 $\mu$m) and calculates the temperature of the object by taking into account emissivity $\varepsilon$ of the same object and other environmental parameters, such as reflected temperature compensation RTC. Typically, the radiation recorded by the thermal camera results from the superimposition of three contributions: emitted radiations $\varepsilon$, reflected radiations p and transmitted radiations $\tau$. The higher is the proportion of transmitted or reflected radiations, the harder is performing a precise measurement of the temperature of an object. In the field of application of the invention, transmitted radiations are substantially of no relevance and reflection factor is low. Instead, emissivity is high and therefore the temperature can be easily measured by means of a thermal camera.

US 2004158156 A1 discloses for instance a device for detecting temperature increase caused by injuries in limbs of horses or other mammalians. The device is equipped with thermo-chromic liquid crystals whose colour changes depending on heat being detected, thereby providing thermal images of the area of interest where the device is applied.

The publication by T.A. Turner, R.C. Purohit, J.F. Fessler, "Thermography: A review in equine medicine", Comp. Cont. Ed. 8, 855, 1986, further reports that infrared thermography could be useful in diagnosing equine lameness. The publication by Weil, M., Litzke, L.F., Fritsch, R. "Diagnostic validity of thermography in equine lameness", Tierarztliche Praxis Ausgabe G: Grosstiere - Nutztiere - Volume 26, Issue 6, 1998, pages 346-354, however reports that thermography in diagnosing equine lameness is useful only in combination with an in-depth clinical examination involving supplementary investigations.

The publication by Simone Westermann, et al. "Effects of infrared camera angle and distance on measurement and reproducibility of thermographically determined temperatures of the distolateral aspects of the forelimbs in horses", Journal of the American Veterinary Medical Association, February 1, 2013, Vol. 242, No. 3, pages 388-395, discloses the effects of changing the positioning angle and distance of a thermal camera relative to horse forelimbs. This document also suggests analysing temperature differences between the limbs.

A.L. Eddy, L.M. Van Hoogmoed and J. R. Snyder "The Role of Thermography in the Management of Equine Lameness", The Veterinary Journal 2001, 162, 172-181, report that, even though thermography is not capable of detecting specific pathologies, yet it facilitates locating temperature increases due to inflammations or injuries, and temperature decreases due to a reduction in circulatory capacity and vasomotory tone.

The studies developed till now for detecting pathologies affecting cattle limbs, such as the studies mentioned above, have a considerable limitation because of the substantial impossibility of application in zootechnical industry. Indeed, they require application under conditions that are hardly reproducible in a zootechnical farm. One of the factors mainly affecting the results of thermographic analysis is the noise due to environment temperature and to the spatial position of the distal part of the animal's limb. The studies at present known are generally based on a comparison between the temperatures of two hoofs, in particular the fore hoofs, of the animal. It has been experimentally demonstrated that significant temperature differences between the hoofs are an indication of the presence of certain pathologies. Yet, the problem of how to obtain thermographic images useful for diagnosing the state of health of the distal part of the limb, under conditions of normal behaviour of the animals in a farm, for instance a dairy cow farm, is not dealt with.

The studies mentioned above moreover are not suitable for providing a significant indication about the onset of a specific pathology sufficiently in advance with respect to the appearance of more clear symptoms in the behaviour of the animal. Moreover the prior art systems are not readily utilisable in environments where farm animals, such as dairy cows, are present, even because such systems are not suitable for tracking an animal that generally is moving. A further drawback of the prior art is related to the fact that the existing systems do not allow detecting pathologies even greatly different from one another.

The publication by M. Alsaood et al. "Detection of hoof lesions using digital infrared thermography in dairy cows", Journal of Dairy Science, Vol. 95, No. 2 (2012), pages 735 - 742, discloses the results of thermographic analyses based on the measurement of the temperature of the skin and the coronary band region of the rear limbs of dairy cows in which lameness was detected. The authors conclude that thermographic analysis can be useful, in association with clinical examination, for detecting lameness resulting from pathological conditions, but they do not propose an apparatus or a method for obtaining, from the images through the thermographic analysis, a diagnosis about the existence and the kind of a possible pathology having caused lameness.

The publication by M. Alsaood et al. "A field trial of infrared tomography as a non-invasive diagnostic tool for early detection of digital dermatitis in dairy cows", The Veterinary Journal 199 (2014), pages 281 - 285, discloses experiments in which thermographic analyses based on the measurement of the temperature of the skin and the coronary band region are carried out and data relating to cows suffering from digital dermatitis and to healthy cows are compared. The authors discuss the problems that can affect the results of the thermographic analysis and conclude that the latter can be useful for an early detection of digital dermatitis, before lameness appearance, but in this case too they do not propose an apparatus or a method for obtaining a reliable diagnosis from the images obtained through the thermographic analysis.

It is an object of the invention to provide a diagnosing system based upon the thermographic analysis of the distal parts of the limbs of quadrupeds, which system is utilisable in a zootechnical industry and allows an early and reliable detection of a plurality of foot pathologies, such as digital dermatitis, digital phlegmon, sole ulcer, white line disease and others.

More particularly, the invention aims at providing a system having a continuous (24 hours/day, 365 days/year) and wholly automatic operation, and allowing obtaining the information required for the diagnosis with a high time resolution.

The above and other objects are achieved by the method, the information technology product and the apparatus as claimed in the appended claims.

*Description of the Invention*

The method of diagnosing pathologies of the distal parts of the limbs of a quadruped, in particular the rear limbs of a dairy cow, is based on the generation of thermographic images of the limbs and the processing of such images. The processing includes the steps of:

- identifying, in each thermographic image and for each limb concerned by the diagnosis, an area containing the distal part, and obtaining from said area an identified image of the distal part;
- validating identified images complying with predetermined geometrical and/or dimensional and/or temperature criteria as images utilisable for diagnostic purposes;
- extracting features significant for the identification of the presence and the kind of pathology from the validated images;
- classifying the distal part of a limb as unaffected by pathologies or as affected by one out of a plurality of pathologies on the basis of such features.

Advantageously, during an observation period, there are generated either a single flow of thermographic images for all limbs to be submitted to diagnosis, or different flows of thermographic images for different limbs. Images relating to a plurality of instants of the observation period are extracted from the or each flow and processed. The diagnosis about the possible presence of a pathology and the kind of same is performed at the end of the observation period, based on the results of the classification performed starting from the images relating to each instant.

Advantageously, the diagnoses relating to a plurality of observation periods can be stored and an alarm signal can be issued when the presence of a pathology has been diagnosed for a predetermined number of consecutive periods.

Preferably, the classification is performed by using machine learning techniques, more particularly the neural network technique.

An apparatus for implementing the method includes one or more thermal cameras for generating thermographic images of the limbs, e.g. a single camera for providing comprehensive images of all limbs concerned or different cameras for providing images of individual limbs, and a processing system programmed to process the thermographic images and to provide, as a result of the processing, a diagnosis about the possible presence of one out of a plurality of pathologies and the kind of pathology. The processing system in turn includes:

- means for identifying, in each thermographic image and for each limb submitted to diagnosis, an area containing the distal part, extracting from said area an identified image of the distal part, checking whether the identified image complies with predetermined geometrical and/or temperature criteria and validating the identified images complying with said criteria as images being utilisable for diagnostic purposes;
- means for extracting features significant for the detection of the presence and the kind of pathology from the validated images;
- classification means for classifying the distal part of a limb as unaffected by pathologies or as affected by a specific pathology on the basis of such features.

The invention also provides an information technology product containing software codes for implementing the method or method steps when the product is loaded into a processing device.

The invention can be advantageously used while a cow is staying in a milking stall. More particularly, the invention lends itself for use in milking installations equipped with a milking barn, let it be of parallel or fishbone or carrousel type, either manual or automated, or in farms equipped with robotised milking. The invention does not oblige the animal to a further forced retention for the diagnostic operations and hence it has no effect on the animal’s stress and does not affect the wellbeing in normal herd management.

However, the invention can be used in any area where cows individually pass and where it is possible to compel a cow to shortly stop to allow taking the limb images.

The diagnosis provided by the invention can be readily associated with a specific animal univocally identifiable in the farm. Actually, as known, in modern farms each animal is monitored by means of devices, such as pedometers or collars, allowing tracing the animal’s habits, and the information provided by the invention can be readily associated with the cow identity provided by other monitoring systems. The invention can therefore become part of a more general monitoring system that enables composing an overall clinical picture of the animal by exploiting multiple indications coming from different sensors and systems, or modifying the animal management profile.

*Brief Description of the Figures*

Some preferred embodiments of the invention will be described by way of non-limiting example with reference to the accompanying Figures, in which:

- Fig. 1 is a schematic side view of a preferred embodiment of the invention;
- Fig. 2 is a block diagram of the system according to the invention;
- Figures 3, 4 show thermographic images of the distal parts of the rear limbs of a dairy cow during milking, said images being taken with a single thermal camera or with two thermal cameras, respectively;
- Fig. 5 shows a frame extracted from the thermographic images;
- Fig. 6 is the flow chart of the processing performed on the thermographic images;
- Fig. 7 is the flow chart of the hoof identification procedure;
- Fig. 8 is a diagram showing the identification result;
- Figs. 9 and 10 are diagrams showing the delayed combination procedure for hoof validation, for a stationary and a rotary milking machine, respectively;
- Fig. 11 shows the raster superimposed onto a hoof image for extracting the features for classification purposes;
- Fig. 12 is a block diagram of a neural network classifier;
- Fig. 13 is a diagram of an alternative classification process; and
- Fig. 14 is a block diagram of the system according to the invention when the classification process shown in Fig. 13 is used.

*Description of some Preferred Embodiments of the Invention*

Referring to Figs. 1 and 2, a preferred embodiment of the invention, applied to diagnosing pathologies of the hoofs of the rear limbs of dairy cows, is schematically shown. Examining the rear limbs only is not a significant limitation, since the pathologies indicated above generally affect always or only such limbs.

The system includes a thermal camera 11 connected to a processing system 13, programmed to convert the thermal signal emitted by the cow and collected by thermal camera 11 into a diagnostic information about an existing pathology, if any.

Thermal camera 11 is associated with a structure 17 for constraining animal 15 into the desired location, e.g. a milking stall or robot for dairy cows, where also a support plane 19 for hoofs $15_{ps}$, $15_{pd}$ of the rear limbs of animal 15 can be provided.

In the embodiment shown, the system includes a single thermal camera 11, positioned so as to simultaneously generate images of the distal parts $15_{ps}$, $15_{pd}$ of both rear limbs of animal 15. Advantageously, thermal camera 11 can be fastened to structure 17 by known means, such as brackets, threaded pins and bolts. In the alternative, thermal camera 11 can be mounted on an own support, for instance a stand, in turn fastened to the floor.

In the embodiment shown, thermal camera 11 is located behind animal 15. The thermal camera is for instance located so that its focus is at a distance of 25 to 70 cm from rear limbs $15_{ps}$, $15_{pd}$ of the animal. Moreover, thermal camera 11 is for instance located at a height of about 30 to 70 cm relative to plane 19 on which rear limbs $15_{ps}$, $15_{pd}$ of animal 15 rest. Axis "S" of thermal camera 11 is therefore for instance inclined by an angle $\alpha$ of about 20° to 70° relative to the vertical direction. Preferably moreover thermal camera 11 is installed so that its longitudinal axis "S" lies in longitudinal plane "PL" of the animal. In such a configuration, axis "S" forms an angle $\beta = 0°$ with plane "PL". Angles $\beta$ in the range -75° to +75° relative to said longitudinal plane "PL" are however compatible with the invention.

In other embodiments, two or more thermal cameras 11 can be provided, e.g. one camera for each limb. In yet other embodiments, one or more movable thermal cameras 11 can be provided, which are e.g. displaceable along a guideway or a track, or are rotatable or yet are mounted on retractile supports, and the or each thermal camera can be brought into position whenever the system is to take an image. The choice of the number and the arrangement of thermal cameras 11 will generally depend on the conditions of use of the apparatus.

The or each thermal camera 11 is arranged to generate a plurality of signals representative of the intensity of the electromagnetic radiation emitted in the infrared range and coming from a plurality of corresponding adjacent areas of the distal part of each rear limb of animal 15. Depending on the kind of thermal camera, a flow of discrete images, a continuous video flow, etc. can be generated. Fig. 3 shows a thermographic image obtained by means of a single thermal camera 11 located behind a cow during milking. In this exemplary embodiment, thermal camera 11 has been located with its axis "S" aligned with longitudinal plane "PL" of the animal. Distal parts $15_{ps}$, $15_{pd}$ of the rear limbs in contact with plane 19 and, in background, milking assembly 12 connected with the animal's teats, are visible in Fig. 3. The same Figure shows the temperature scale in °C. Fig. 4 shows instead a thermographic image obtained by using two low-resolution thermal cameras. In that Figure, the portions within boxes denote a pair of identified and validated hoofs. The manner in which identification and validation are performed will be disclosed hereinafter.

Returning to Fig. 2, processing system 13 is entrusted with the following functions:

- physical interface with thermal camera 11 (connection 14), typically through an Ethernet interface; connection 14 could also be a wireless connection;
- execution of network protocols for communication with the thermal camera (e.g. Real Time Service Protocol, RTSP);
- execution of the algorithms for decoding the video flow(s) provided by thermal camera(s) 11, so as to decompose such flow(s) into a sequence of frames, relating to different instants of an observation period (session), which can be individually analysed; one such frame is denoted 30 in Fig. 5;
- processing of frame 30, including:
    - thermal mapping of the video flow and hoof identification;
    - hoof validation, extraction and interpolation;
    - calculation of indexes significant for the diagnosis;
    - classification of the hoof pair and diagnosis;
    - graphical display of the results on a screen and saving of such results into a data base accessible to the system itself and eventually from the outside of the system.

In order to perform such operations, system 13 includes the actual processing device 20, which may consist of a single unit performing the whole processing from video frame extraction to diagnosis, by using one or more internally stored programs, or may include multiple units, each performing a part of the processing. For instance, inside block 20, reference numerals 20A, 20B, 20C and 20D denote processing units or program routines performing frame extraction, hoof identification and validation, feature extraction and classification, respectively. System 13 further includes a first buffer 16 temporarily storing the results of all classifications performed for a same hoof pair during a session, for instance during the period the cow is staying in milking station 17, and a decision unit 21 that provides the diagnosis on the basis of the stored classifications. For instance, unit 21 can be a majority logic network. A second buffer 22 allows temporarily storing the validated images, for purposes that will be disclosed below.

Decision unit 21 sends the diagnosis to the storage device accommodating the data base and to the display device, said devices being jointly schematised by block 18.

The following data can be for instance stored in the data base:

- final diagnosis;
- diagnosis time;
- snapshot of the left and right hoofs;
- maximum temperature of the left and right hoofs;
- confidence of the diagnosis, based on the distribution of the provisional diagnoses.

Moreover, if the invention is included into a general monitoring system of the farm, such data can be associated with the animal's identity and integrated with information coming from other devices, for instance collars, pedometers, etc., in order to contribute to build a more complete clinical picture, or to allow interventions on the management of the individual animal.

Decision unit 21 can also be associated with a storage and signalling device 23 that receives the diagnoses issued by decision unit 21 and generates an alarm signal when a specific disease has been diagnosed for a predetermined number of consecutive observation periods. In this way, the number of false positives is reduced.

Referring to Fig. 6, the processing leading to the diagnosis is disclosed in greater detail.

Step 101 is the image decoding and frame extraction from the flow(s) coming from the thermal camera(s), and it is carried out by using wholly standard algorithms, well known to the skilled in the art of image processing: the choice will obviously depend on the video flow format (for instance, H264, MPEG, MJPEG...). Preferably, the frames extracted are stationary images. If multiple video flows are generated, step 101 is performed for each flow, and the frames simultaneously extracted are combined into a single composite image on which the subsequent processing is performed: for instance, if two different flows are provided for the two hoofs, the frames relating to the individual hoofs extracted at a given instant will be juxtaposed as left and right halves of a single fictitious image containing the hoof pair (see Fig. 4). Frame extraction may take place with a high time resolution, typically about ten frames per second.

System 13 continues analysing the flow of extracted frames in order to detect the presence of an animal (step 102). This presence is signalled by a significant temperature variation in certain frame portions.

Once the presence of the animal has been detected (positive outcome of the check at step 102), the process proceeds to the identification and validation of hoofs $15_{ps}$, $15_{pd}$ (step 103).

For the identification, it is first to be distinguished whether a point of the frame being processed actually belongs to cow 15 or is part of the environment. Different approaches are possible.

A first approach is based on calculating the average temperature of frame 30 (or even of only a portion thereof, shown by box 31 in fig. 5) and separating what belongs to the animal from what belongs to the environment, by selecting a suitable threshold with respect to the average temperature. Points where the temperature is at least equal to the threshold are considered as belonging to the animal, whereas points with lower temperature are considered as belonging to the environment.

A second approach is based on building a histogram of the temperatures inside frame 30 (o portion 31 thereof), whereby minimum value $T_{MIN}$ in the histogram is associated with the environment temperature, whereas maximum value $T_{MAX}$ is associated with the animal's temperature. Based on the minimum and maximum temperature values, it is possible to define an adaptive threshold related with such values and, similarly to the first approach, to consider points where the temperature is at least equal to the threshold as belonging to the animal, and points where the temperature is below the threshold as belonging to the environment.

The third approach is similar to the second one, yet the histogram building and the adaptive threshold calculation are separately performed for both halves of a frame, in order to better take into account the different temperature statistics possibly present in the two limbs.

The second and third approaches are the preferred ones, in case of a frame obtained from a single video flow and in case of juxtaposed frames, respectively.

Fig. 7 shows the identification process in the case of the second approach.

Initial steps 201, 202 are the temperature histogram building and the adaptive threshold definition. Taking into account the concerned temperatures, temperature intervals of the order of a few degrees, e.g. 5°C, and about ten intervals can be considered for building the histogram. As to threshold $T_S$, in the preferred embodiment it is given by relation:

$$T_S = T_{MIN} + (T_{MAX} + T_{MIN})/K$$

where K is a parameter that has been selected in the range 2 to 4. Such a range has generally proven valid for the temperatures of the environment and the animal encountered during milking operations.

At this point, the process can proceed to identifying the rear hoofs in the image portion belonging to the animal. To this end, frame 30 or portion 31 thereof are scanned from the bottom upwards in order to search for the first transition point from a condition below the threshold to a condition above the threshold (step 203). Referring to Fig. 5, where frame 30 contains right front hoof $15_{ad}$ besides right rear hoof $15_{pd}$, the scanning identifies the latter. Once said point has been found, the end points of the region where temperature remains at least equal to the threshold are searched for at the right and the left of that point, thus obtaining a segment 32 defining the width of the lower hoof portion (step 204). By using such a segment, a polygon is built defining the useful hoof area (step 205, hoof framing). Preferably, the polygon is a quadrilateral, for instance a square whose side is equal to that segment, obtained by shifting segment 32 upwards, as shown by the vertical segments in Fig. 5. The square built in this manner is denoted 33 in Fig. 8, where the hoof is seen from a different angle with respect to Fig. 5, and it forms the identified image that is extracted and submitted to validation (step 206).

In case of the third approach, the process shown in Fig. 7 is simultaneously performed on both image halves.

Returning to Fig. 6, once the alleged hoofs have been identified, validation thereof is performed, i.e. it is checked that the alleged hoofs comply with certain criteria relating to the size, the position and the temperature characteristics in order the subsequent diagnostic formulation is reliable. To the same end, it is also necessary to check that the hoofs are shot by the thermal camera under suitable conditions.

In a preferred embodiment of the invention, validation is performed in two steps: in a first step, it is checked that what has been identified as a hoof by means of the previous operations is actually a hoof, and in a second step it is checked that the hoof identified is utilisable for the diagnostic operations.

In a preferred embodiment of the invention, the criteria used in the first step are as follows:

1) <u>Temperature difference criterion</u>

Such a criterion is based on the assumption that a pixel column, corresponding to the limb, with temperature not significantly lower than the hoof temperature, is to correspond to each actual hoof. Otherwise, the area being considered is not a hoof, but a hot area related for instance to dejections, parts of the milking apparatus and so on.

2) <u>Minimum/maximum temperature criterion</u>

Such a criterion is based on the assumption that, even if the condition of exceeding the environment/animal threshold is met for a group of pixels, if the square built in correspondence of the hoof includes at least one point having a temperature not compatible with the cattle biology, then the area being considered is not a hoof.

3) <u>Maximum size criterion</u>

Such a criterion is based on the assumption that, due to the features of thermal camera installation in milking station 17, the hoof cannot have a size exceeding a certain threshold typical of the particular installation.

4) <u>Criterion of appurtenance to a defined area</u>

The application conditions of the thermal camera in milking station 17 may involve that it is known in advance that, in certain areas of the thermographic image, no hoof can be present or elements can appear that could be erroneously interpreted as hoofs. An example of such a condition is the presence of the tubes conveying the milked milk, generally located centrally in the image, since the freshly milked milk can originate temperature features meeting the temperature criteria mentioned above.

In a preferred embodiment of the invention, the criteria used in order hoofs having complied with the criteria discussed above can be considered as acceptable are as follows: I. <u>Minimum size criterion</u>

An accurate diagnosis requires a minimum amount of information to be extracted from the hoof cut from the whole thermographic image. If the size of the hoof identified does not comply with such a condition, it is preferred to consider the hoof as unsuitable for the formulation of a diagnostic hypothesis and to wait for a hoof complying with the requirements, rather than to go on with a processing whose result could be unreliable. For instance, in case of a resolution 60 x 80 pixels, a hoof size of 12 to 25 pixels is considered acceptable.

II. <u>Criterion of distance from the edge</u>

In certain cases, the hoof could be located near the bottom or the side edge of the image. In such cases, the hoof could be not wholly included in the thermographic image. Also in this case, it is preferred to consider the hoof as unsuitable for the formulation of a diagnostic hypothesis if the minimum distance of the alleged hoof from the edge does not exceed a given threshold.

III. Angle criterion

When a hoof is detected, it is associated with an angle estimating the hoof inclination with respect to a hypothetical axis vertically oriented relative to the ground. The angle is calculated as the angle included between the middle point of the bottom hoof edge and the middle point of the top hoof edge (see Fig. 8, line 34). When the angle is excessive, the diagnostic accuracy can be jeopardised and hence the identification is considered unreliable. On the other hand, Fig. 8 shows that taking into account the hoof inclination allows including into the identified hoof even regions, such as region 15p', that would be ignored if the area initially obtained by means of square 33 would be considered.

A back-correction of the thermographic image by taking into account the angle measured could also be possible, but this approach is considered sub-optimal with respect to the simple rejection of excessively inclined images. Indeed, given the availability of a great amount of images from the thermal camera, it is preferred to take into account only the correct images, even if possibly they are a minority fraction, rather than to jeopardise the diagnostic accuracy.

In an embodiment of the invention, the above criteria are to be simultaneously complied with for both hoofs in order the validation takes place.

Yet, in some applications, a simultaneous validation can be difficult to obtain. For instance, in a rotary milking system, it may happen that one hoof is not shot at a certain instant. Similarly, in a stationary milking system, even if the thermal camera simultaneously shoots both hoofs, it may happen that only the image of one of them complies at a certain instant with the validity criteria. In order to take this into account, in the preferred embodiment, whenever a hoof has been validated, the image thereof is temporarily stored in buffer 22, in separate queues for the right and left hoofs. If the validated images of the hoofs of both limbs are simultaneously available, they are used for the subsequent classification and are deleted from buffer 22. If the hoof of only one limb has been validated or is available, the image thereof is kept in buffer 22 while awaiting the availability or validation of the hoof of the other limb. At that moment, the images of both hoofs are combined and the process goes on as if the images had been obtained at a same instant (delayed combination). This procedure allows reducing the number of samples to be processed in order to arrive at the diagnosis.

The delayed combination procedure is schematically shown in Figs. 9 and 10 for a stationary and a rotary milking machine, respectively, for a number of observation instants t1...t5. Solid line squares represent valid hoofs, and broken line squares represent invalid hoofs.

In Fig. 9, at instant t1, only the right hoof is valid and its image 1D is stored in right queue 22D, whereas image 1S of the left hook is neglected. The same situation occurs at instant t2, whereby queue 22D will contain images 1D and 2D. At instant t3, both hoofs are valid: their images 3S and 3D are stored into the respective queues and used by index extraction unit 20C in processing device 20, which deletes them from the queues after use. Use and deletion are denoted by the double line arrow. After such an operation, buffer 22 still contains images 1D and 2D in queue 22D. At instant t4, only the left hoof is valid and its image 4S is stored into left queue 22S. Such an image is then associated with image 2D available in queue 22D, thereby forming a pair that, as before, is used for index extraction and then deleted from buffer 22, where image 1S still remains. At instant t5, again, only the left hoof is valid and its image 5S is stored into the left queue and associated with image 1D for use.

In case of a rotary milking machine, the operation is wholly similar. Yet, it is to be taken into account that at some instants (for instance t1, t4 and t5) a hoof can be missing or the right hoof can be located in the left half of the frame (instant t1) or vice versa (instants t4, t5). Anyhow, missing and invalid hoofs are handled in the same manner. Moreover, device 20 is programmed so as to detect which hoof is located in a given half frame, so that, if the hoof is valid, the image can be stored in the proper queue.

Once the validation is over, extraction of the features or indexes to be used for the classification is performed (Fig. 6, step 105).

In this respect, it is to be taken into account that, in general, the hoofs identified have an unknown and variable size, depending on the hoof shape, size and distance from the thermal camera. Yet, in order the diagnosis can be reasonably accurate, it is better to work onto images having the same sizes, measured as number of pixels. It is therefore necessary to carry out a resampling or interpolation of the hoof image so that the image size can be brought to a number of pixels identified as standard. That standard number of pixels has to be not lower than the number of pixels potentially present in the starting image, in order no useful information is destroyed. When using a thermal camera with resolution 60x80 pixels, the starting image is typically 25x25 pixels and, in practice, the standard hoof size will be at least 32x32 pixels (it is preferred to operate with sizes that are powers of 2). Even though obviously an interpolation operation does not add information, it has been noted that the resolution increase by mathematical way allows obtaining better diagnostic accuracies. Taking this into account, the size of the hoof extracted from the starting image is preferably standardised to 64x64 pixels, whichever the hoof starting size: a value of this kind does not significantly add to the processing burden.

A raster 35 (Fig. 11) is then created on the thermographic image of each hoof, interpolated to 64x64 pixels, so as to obtain a plurality of areas or cells. A set of indexes or features, which are significant for the classification to be performed and which are chosen so as to allow efficiently exploiting the available computational resources, will be extracted from each cell. For the sake of simplicity of the drawing, a square raster made of square cells has been shown in the Figure: in general, however, the cells in the raster will be polygonal and will have different areas. The number of cells in the raster is chosen by taking into account two opposite requirements:

- few cells do not allow a sufficient spatial resolution of the hoof; and
- too many cells make the algorithm computationally more burdensome and tend to cause a loss of generality of the indexes extracted, since the association of an index with adjacent cells will suffer from the uncertainty introduced by the animal shape and the shooting angle.

The nine cells shown in the drawing are a satisfactory compromise.

The bottom, left and right edges of raster 35 can for instance correspond with the bottom, left and right edges of square 33 in Fig. 8, and the raster height can be predetermined so as to accommodate hoofs with different sizes. Moreover, since rear hoofs $15_{ps}$, $15_{pd}$ generally are not arranged perpendicularly to thermal camera 11 while the thermographic images are being taken, it is necessary to correct the inclination of raster 35 in the third dimension, i.e. the depth, in order to obtain a correct positioning of raster 35 relative to the actual positioning of the distal parts of the limbs. This is carried out by making the bottom side of raster 35 coincide with the temperature difference line and by correcting the axonometric offset according to a known algorithm, depending on the inclination the bottom side of raster 35 will have relative to the horizontal axis. Such a correction is made for each rear hoof $15_{ps}$, $15_{pd}$ of the animal.

Fig. 11 further shows that a safety band 36, from which no index will be extracted, exists around raster 36. The reason for the presence of such a band is that disturbing elements may be present at the interface between the hoof and the external environment: those elements may have several causes, such as the presence of biological material under and by the side of the hoof and uncertainty in the angle estimation (the hoof inclination could be greater than estimated), and might cause wrong diagnoses.

In a preferred embodiment the indexes extracted are:

- absolute temperature indexes, which synthetically consider the minimum, maximum or average temperature for the cell being considered;
- temperature variation indexes, which take into account the thermal variability in the cell being considered, e.g. variance or entropy;
- temperature distribution indexes, which are obtained by comparison (or difference) of other temperature indexes in areas astride different cells in the same hoof;
- indexes obtained as difference between homologous indexes in left and right hoofs $15_{ps}$, $15_{pd}$; in most cases, the presence of a pathology, both an infective and a biomechanical one, gives rise to an asymmetry between the left and right hoofs, both because generally only one hoof is affected, and because, if both hoofs are affected, it is difficult that the pathology identically affects them, so that a difference between the hoofs is generally very significant;
- motion indexes, based on the analysis of the hoof coordinates in the image; such indexes exploit the well-known fact, exploited by diagnostic methods based upon the observation of the animal's behaviour, that a relation exists between the number and the type of hoof movements on the one side and the existence of a pathology on the other side.

By using the indexes extracted at step 105, the process proceeds to the classification (step 106), which preferably is performed by using machine learning techniques, in particular the neural network technique. In the case of diagnosis of the four pathologies mentioned above, each hoof is allotted one of five classes, e.g. class 0 = healthy hoof, and classes 1, 2, 3, 4 = hoof affected by digital dermatitis, digital phlegmon, sole ulcer and white line disease, respectively. Step 106 hence provides a diagnosis forecast at each instant of the observation period.

Steps 104 to 107 are automatically repeated several times while the animal is staying in observation location 17 (positive outcome of the new check on the presence of the animal, step 108). For instance, taking into account that a cow remains inside the milking robot for a time period of 6 to 8 minutes, the cycle can be repeated 10 to 50 times during such a period. At the end of the session, when cow 15 leaves location 17, decision unit 21 reconsiders all provisional diagnoses (forecasts) and, based on the distribution thereof and on other criteria, provides a final diagnosis (step 109). The simplest example of how this can be made is using a majority decision unit 21 that counts the number of occurrences of each of the five output values of the classifier and eventually selects the value with the highest number of occurrences.

Of course, it would also be possible to issue the diagnosis while the animal still is in the milking station, after a predetermined number of iterations.

Fig. 12 shows a preferred embodiment of classifier 20D (Fig. 2) when the neural network technique is used. Classifier 20D is organised on two levels, of which the lower (or slave) level responds to the need of a high specificity in order to detect a plurality of pathologies (which demands many training sessions), whereas the upper (or master) level responds to the need of keeping a sufficient generality, which demands a reduced number of training sessions.

More particularly, the slave level includes, in the embodiment shown, three neural networks 41, 42, 43, which receive the indexes extracted in step 105 (Fig. 6) and provide three classifications with different fineness degrees. More particularly:

- network 41 assigns the hoofs to one of two classes: healthy hoof (e.g. class 0), or hoof affected by a pathology (class 1);
- network 42 assigns the hoofs to one of three classes: healthy hoof (class 0), hoof affected by an infective pathology, e.g. digital dermatitis or digital phlegmon (class 1), hoof affected by a biomechanical pathology, e.g. sole ulcer or white line disease (class 2);
- network 43 assigns the hoofs to one of the five classes 0, 1, 2, 3, 4 mentioned above

Master level includes a single network 44 that receives both the indexes and the classification results provided by each slave network 41 - 43 and acts as an arbiter among the slaves, providing a diagnosis forecast relative to the current cycle that is stored in buffer 16 (step 107).

Table 1 below shows by way of example the results of three subsequent classifications as provided by classifier 20D during a field trial of the invention. In this trial, two thermal cameras 11 have been used, since the features of the milking robot with which the invention was associated did not allow satisfactorily taking images of both limbs by means of a single camera. The resolution of thermal cameras 11 was 60x80 pixels and, as said, hoof sizes of 12 to 25 pixels have been deemed acceptable.

TABLE 1

| Classification counter | Classification vector | Network 44 | Network 43 | Network 42 | Network 41 | Diagnosis forecast |
|---|---|---|---|---|---|---|
| 57 | [53, 0, 0, 4, 0] | 0 | 0 | 0 | 0 | 0 |
| 58 | [54, 0, 0, 4, 0] | 0 | 0 | 0 | 1 | 0 |
| 59 | [54, 0, 0, 5, 0] | 3 | 3 | 1 | 1 | 3 |

Column "Classification counter" shows the number of classification cycles performed up to that instant.

Column "Classification vector" shows the distribution of the results among the five classes provided for.

Columns "Network 44" etc. show the class allotted by each neural network to the hoof under examination in that particular cycle.

Column "Diagnosis forecast" shows the final classification in each cycle (which in the example coincides with the classification allotted by network 44).

The Table shows that successive classifications generated by the classifier may be different and may be differently generated. For instance, the same class "0" is obtained at cycle 57 with the agreement of all three slave networks, and at cycle 58 with a partial disagreement among them. Also in the case of cycle 59 a disagreement occurs among the three slave networks, yet, contrary to cycle 58, the classification decided by network 44 does not coincide with that of the majority of the slave networks.

The preceding description clearly shows that the invention provides a method and an apparatus that are non-invasive, cheap and readily applicable in zootechnical industry, have a continuous operation and allow obtaining in wholly automatic manner a reliable diagnosis of the possible presence of pathologies in quadrupeds. Given its non-invasive character, the invention can be repeatedly and frequently employed, and this makes possible an early diagnosis of the onset of pathologies.

It is also clear that the invention can be applied without difficulties to the diagnosis of other pathologies giving rise to thermal anomalies in the distal parts of cattle limbs. Moreover, even if only the rear limbs are of interest for diagnosing the cattle pathologies mentioned here by way of example, in case of other pathologies and other farm animals the method and the apparatus described can be used for diagnosing pathologies also or only in the fore limbs, by suitably arranging the thermal cameras.

It is also to be appreciated that, even if the use of the invention in a milking barn has been mainly described hereinbefore, the or each thermal camera 11 can be located in any area of the farm where cows individually pass, for instance in the passage corridor used for controlling the afflux to the milking barn. In this case, means will have to be provided for compelling the cow to a short stop, to allow taking the limb images. For instance, a timed output gate could be provided, which opens after a predetermined time such as to allow the acquisition of a number of frames (by way of indication only, assuming a time resolution of 10 frames per second, such time might be a few seconds). The gate will anyway open after the predetermined time has elapsed, even if frame acquisition did not occur. Anyhow, generally speaking, it is possible to create an ad hoc structure, with which the apparatus of the invention can be associated, for temporarily constraining individual animals. This clearly allows a more general use of the invention. It is to be appreciated that, in case of short stays of the animal in the observation area, the delayed combination procedure for hoof validation can be of particular interest.

Moreover, even if a fixed installation has been described above, the invention readily lends itself to make a portable device, thanks to the availability on the one side of thermal cameras associable with smart phones with different operating systems (for instance, thermal cameras Flir One or Seek Compact Thermal Imager) and on the other side of smart thermal cameras, i.e. thermal cameras provided with substantial processing capabilities (e.g. thermal camera Argus Mi-TIC-E). A portable, pocket-sized device by which a diagnostic analysis can be performed whenever the need arises is clearly very attractive.

It is clear that the above description has been given only by way of non-limiting example and that changes and modifications are possible without departing from the scope of the invention as defined by the appended claims.

For instance, for hoof identification, known algorithms for detecting objects in computerised images, such as the Viola-Jones algorithm for human face detection, could be used in place of the algorithm disclosed with reference to Fig. 7. Yet the algorithm described here has the advantage of a far lower computational burden (about 1/100 that of the Viola-Jones algorithm).

Moreover, a classifier 20D based on neural networks, instead of including networks organised on two levels as described, could include a plurality of networks organised on a single level and take a majority decision. As diagnosis forecast at a given instant, the forecast on which a predetermined number of the networks agree will be provided: e.g., five networks could be used and the diagnosis forecast provided could be that provided by at least four networks.

Moreover, classifier 20D, instead of being based on neural networks, could be a classifier based on different machine learning techniques, with or without supervision, such as an SVM, Bayes, Boost, Bagger Tree classifier or the like.

Furthermore, the indexes of interest for the diagnostic formulation, instead of being processed according to machine learning techniques, could be processed by means of logic operations explicitly expressed, as disclosed hereinbelow with reference to Figs. 13 and 14. In the latter Figure, elements corresponding to those shown in Fig. 2 are denoted by the same reference numerals.

The image portions corresponding to hoofs $15_{ps}$, $15_{pd}$ of the animal, after having been validated, are still enclosed by processing device 20 into corresponding cell rasters 35A, the position of which is set in similar manner to what disclosed with reference to raster 35, by determining temperature transition zones, i.e. zones where a significant temperature difference, typically at least 1°C, is detected.

Preferably, rasters 35A and the individual cells have square or rectangular shape, and advantageously the cells have sizes lower than the thermal camera resolution. It is sufficient that each cell includes at last one pixel. For instance, for a thermal camera with resolution 320x220 pixels, each cell in the raster could contain 10x10 = 100 pixels. When using a thermal camera with resolution 60 x 80 pixels, as indicated for the previous embodiment, the number of pixels per cell can be reduced accordingly. Raster 35A could for instance include 18x18 cells. The cells in raster 35A are identified through their spatial coordinates on axes X and Y.

For classification, temperature $T_{x,y}$ measured in the generic cell with coordinates x, y inside raster 35A is compared in device 20 with a temperature threshold $T_{s1}$ and, depending on the outcome of the comparison, the hoof is assigned to either of two classes: healthy hoof (e.g. class 0), if $T_{x,y} \leq T_{S1}$, or hoof affected by a pathology (class 1), if $T_{x,y} > T_{s1}$. Value $T_{x,y}$ can be the temperature value measured at the cell centre, or any temperature value measured in the cell, or yet the average temperature value in the cell. Threshold $T_{S1}$ can be the same for all cells in the raster, or it is possible to set different threshold values depending on the cells and hence on the spatial coordinates of the cells in raster 35A. Preferably, threshold $T_{S1}$ is set to value $T_M + T_K$, where $T_M$ is the measured temperature and $T_K$ is a correcting factor. $T_M$ may be the ambient temperature $T_A$ and correcting factor $T_K$ is in the range 3 to 20°C and more preferably is about 9°C. As an alternative, $T_M$ is skin temperature $T_C$ of animal 15 under observation and correcting factor $T_K$ is in the range 10°C to 30°C and more preferably is about 6°C. Both ambient temperature $T_A$ and skin temperature $T_C$ could even be measured. In the latter case, two temperature thresholds $T_{S11}$ and $T_{S12}$ will be provided, and threshold $T_{S1}$ is obtained as the average of thresholds $T_{S11}$ and $T_{S12}$. Threshold $T_{S1}$ could even have a fixed value, for instance 22°C. Reference numeral 25 in Fig. 14 denotes a probe for measuring $T_A$ or $T_C$ (or a pair of probes, if both temperatures are measured), suitably connected with processing device 20.

Spatial coordinates x, y of the cells in raster 35A where threshold $T_{S1}$ has been exceeded are also compared with spatial coordinates x, y of cells denoting the presence of a particular pathology that may affect the distal part of the animal's limb. The spatial coordinates of those cells are stored in a storage unit 26, e.g. a programmable read only memory, accessible by processing device 20. In storage unit 26 the cells are grouped into cell groups or strings, each associated with a specific pathology W0, W1, W2...Wn. A same cell may belong to multiple strings, yet the strings will differ by at least one cell. Strings W0...Wn associated with the homonymous pathologies are stored into unit 26 in a programming step of same. When the coordinates of the cell where $T_{x,y} > T_{S1}$ correspond with the coordinates of one of the cells of the strings stored in unit 26, device 20 classifies the hoof as affected by pathology W0...Wn.

For instance, Fig. 13 shows six zones corresponding to strings W0...W5. String W0 defined by a dashed-and-dotted line is associated with digital dermatitis. Strings W1 and W2 enclosed within a perimeter marked by a dotted line are associated with white line disease. Strings identified by the overlap of perimeters W0 and W1, W2 are associated with phlegmon disease. Strings W3 and W4 enclosed in a perimeter marked by a broken line are they too associated with white line disease. String W5 defined by a line of symbols "+" is associated with sole ulcer.

The coordinates of a cell where threshold $T_{S1}$ has been exceeded may also be processed jointly with the coordinates of the other cells where $T_{x,y} > T_{S1}$. Those "positive" cells form cell strings that are compared with the cell strings associated with pathologies W0...Wn and stored in storage unit 26. In such case, in unit 26, the coordinates of such cells are also associated with an index or weight of the degree of disease development. Processing device 20 is programmed so as to sum the weights associated with the coordinates of the cells in which temperature threshold $T_{S1}$ has been exceeded and which correspond to cells stored in unit 26, and to signal both the pathology detected and the development degree thereof, the latter being proportional to the sum of the weights of the cells in unit 26 for which the correspondence has occurred and which belong to the string associated with the pathology.

The processing of the classifications generated in this manner in order to arrive at the diagnosis takes then place as described above.

## Claims

1. An automatic method of diagnosing pathologies of the distal parts ($15_{ps}$, $15_{pd}$) of the limbs of a quadruped (15), comprising the operations of:

   - generating thermographic images of said limbs;
   - processing (101 - 109, 201 - 206) the thermographic images;
   - providing (109), as a result of the processing, a diagnosis on the presence, if any, of one out of a plurality of pathologies and, in case the presence of the pathology is diagnosed, on the kind of pathology;

   the method being **characterised in that** the processing operation includes the following steps:

   - identifying (103, 104), in each thermographic image and for each limb concerned by the diagnosis, an area containing said distal part ($15_{ps}$, $15_{pd}$), and forming an identified image of the distal part from said area;
   - validating identified images complying with predetermined criteria as images utilisable for diagnostic purposes;
   - extracting (105) features that are significant for the detection of the presence and the kind of pathology from the validated images;
   - classifying (106) the distal part of a limb as unaffected by pathologies or as affected by said one out of a plurality of pathologies on the basis of such features.

2. The method according to claim 1, wherein:

   - the operation of generating thermographic images is performed during an observation period in which the animal (15) stays in an observation location (17) and generates either a single discrete or continuous flow of thermographic images for all limbs to be submitted to diagnosis, or different discrete or continuous flows of thermographic images for different limbs;
   - the processing operation is arranged to extract images relating to a plurality of instants of the observation period from the or each flow, images extracted at a given instant from different flows being combined into a single resulting image on which identification of the distal parts of all limbs is performed;
   - the diagnosis about the possible presence and the kind of pathology is performed at the end of the observation period, based on the results of the classification performed starting from the images relative to said plurality of instants.

3. The method according to claim 1 or 2, wherein the step of identifying the image of a distal part includes the steps of:

   - calculating an average temperature of each image or of a portion of each image;
   - identifying image points where the temperature exceeds the average temperature as belonging to the distal part;

or, in the alternative, includes the steps of:

- creating (201) at least one histogram of the temperature in each image or in a portion of each image;
- generating (202) at least one adaptive temperature threshold;
- defining (203, 204) end margins of the image or portion of the image of the distal part ($15_{ps}$, $15_{pd}$) by identifying points where a transition occurs from a temperature condition above the threshold to a temperature condition below the threshold and vice-versa;
- creating, starting from such points, a framing polygon defining a useful area of the distal part.

**4.** The method according to any preceding claim, wherein the validation step (104) includes a check on the compliance with at least one of the following criteria or with a combination of at least some of the following criteria:

- size of the distal part ranging from a minimum to a maximum size;
- minimum and maximum temperatures in the distal part lying in a range compatible with the animal's biology;
- distance from the image edges not lower than a given minimum distance;
- inclination of the distal part not exceeding a given angle.

**5.** The method according to any preceding claim, wherein the step of feature extraction includes the steps of:

- dividing a validated image into a plurality of contiguous cells belonging to a raster (35; 35A); and
- extracting the features from each contiguous cell;

and wherein the classification is performed, at a given instant, by using features extracted from cells of a validated image relating to at least one limb.

**6.** The method according to any preceding claim, wherein the features extracted from each cell include:

- absolute temperature features;
- temperature variation features;
- temperature distribution features, obtained from a comparison with other cells of the same distal part;
- features obtained as a difference between homologous features in the different distal parts;
- features of motion of the distal part;

and wherein the step of feature extraction from the images is performed after having interpolated said images to a predetermined standard size.

**7.** The method according to any preceding claim, wherein the classification step is performed by means of machine learning techniques, more particularly by means of the neural network technique.

**8.** The method according to any of claims 1 to 5, wherein the features extracted from each cell include a temperature measured within the cell, and the classification step includes the steps of:

- comparing the temperature measured in each cell with a predetermined temperature threshold, and classifying the distal part of the limb as unaffected by pathologies if the temperature measured does not exceed the threshold, otherwise classifying it as affected by a pathology;
- defining cells of the raster (35A) indicative of the onset of one out of a plurality of pathologies, and grouping said cells into first strings (W0... Wn) in one to one association with a specific pathology;
- comparing spatial coordinates (x, y) of the cells in which exceeding of the temperature threshold ($T_S$) has taken place with spatial coordinates (x, y) of the cells of said first strings (W0...Wn) and, in case of positive outcome of the comparison, classifying the distal part of the limb as affected by the specific pathology;

and wherein moreover the cells of said first strings (W0...Wn) are optionally associated with an index of the development degree of the pathology, and the classification step includes the steps of:

- grouping the cells in which exceeding of the threshold has been detected into second strings of cells, and comparing said second strings with the first strings;
- in case of positive outcome of the comparison, summing the indexes of the first strings for which the comparison has given positive outcome together in order to determine a development degree of the pathology; and

- associating the development degree of the pathology with the classification.

**9.** The method according to any preceding claim, further comprising the operations of:

- storing the diagnoses generated in a plurality of consecutive observation periods into a memory; and
- signalling the existence of a pathology when the latter is diagnosed in a predetermined number of consecutive observation periods.

**10.** An information technology product utilisable by a processing system and containing program codes readable by said system for implementing the method according to any preceding claim.

**11.** An automatic apparatus for diagnosing pathologies of the distal parts (15ps, 15pd) of the limbs of a quadruped (15), comprising:

- at least one thermal camera (11) for generating thermographic images of said limbs;
- a processing system (13) programmed for processing the thermographic images and for providing, as a result of the processing, a diagnosis on the presence, if any, of one out of a plurality of pathologies and, in case the presence of the pathology is detected, on the kind of pathology;

**characterised in that** the processing system (13) includes:

- means (20B) for identifying, in each thermographic image and for each limb concerned by the diagnosis, an area containing said distal part ($15_{ps}$, $15_{pd}$), extracting from said area an identified image of the distal part, and validating identified images complying with predetermined criteria as images utilisable for diagnostic purposes;
- means (20C) for extracting features significant for the detection of the presence and the kind of pathology from the validated images;
- classification means (20D) for classifying the distal part of a limb as unaffected by pathologies or as affected by a specific pathology on the basis of such features.

**12.** The apparatus according to claim 11, comprising either a single thermal camera (11) for all limbs to be diagnosed or different thermal cameras (11) for different limbs to be diagnosed, and wherein:

- the or each thermal camera (11) supplies the processing system (13) with a discrete or continuous flow of thermographic images generated during an observation period; and
- the processing system (13) includes:

  - means (20A) for extracting from the or each flow and processing images relating to individual instants of the observation period;
  - a first storage unit (16) for temporarily storing the classification results relating to each said instant; and
  - a decision unit (21) arranged to provide the diagnosis about the possible presence and the kind of pathology at the end of the observation period, based on the results of the classifications performed during the observation period.

**13.** The apparatus according to claim 11 or 12, wherein:

- the identification and validation means (20B) are associated with a second storage unit (22) for temporarily storing, into a separate queue (22S, 22D) for each limb, validated images of the distal parts relating to different instants of the observation period; and
- the feature extraction means (20C) are arranged to extract the features either from validated images relating to a same instant for all limbs, or from validated images relating to different instants for different limbs, depending on whether or not the validation relating to a certain instant of the observation period has been successful for all limbs, the feature extraction means (20C) commanding the deletion from the second storage unit (22) of the images from which the features have been extracted.

**14.** The apparatus according to any of claims 11 to 13, wherein the classification means (20D) include logic networks operating according to machine learning techniques, more particularly neural networks (41 - 44).

**15.** The apparatus according to any of claims 11 to 14, wherein the processing system (13) further includes a storage and signalling unit (23) for storing the diagnoses issued by said decision unit (21) and generating a signalling notifying the existence of a pathology when the pathology has been diagnosed for a predetermined number of consecutive observation periods.

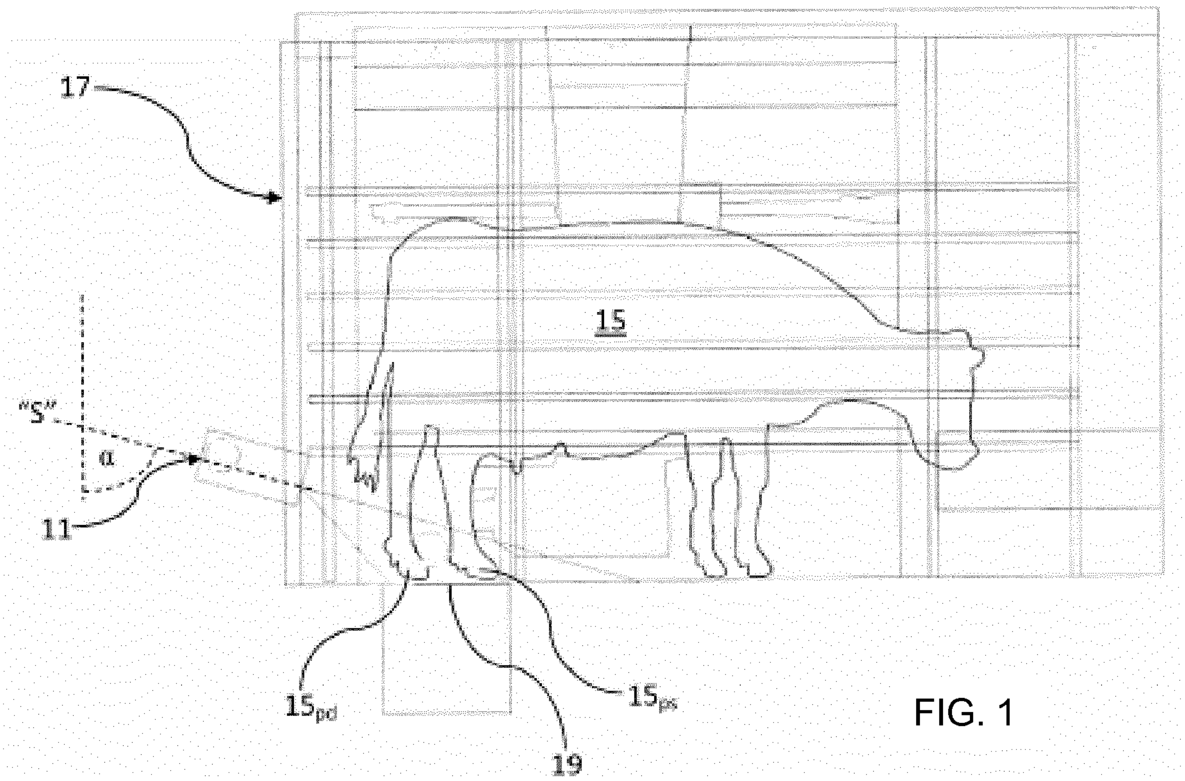
17
15
"S"
α
11
15pd
19
15ps

FIG. 1

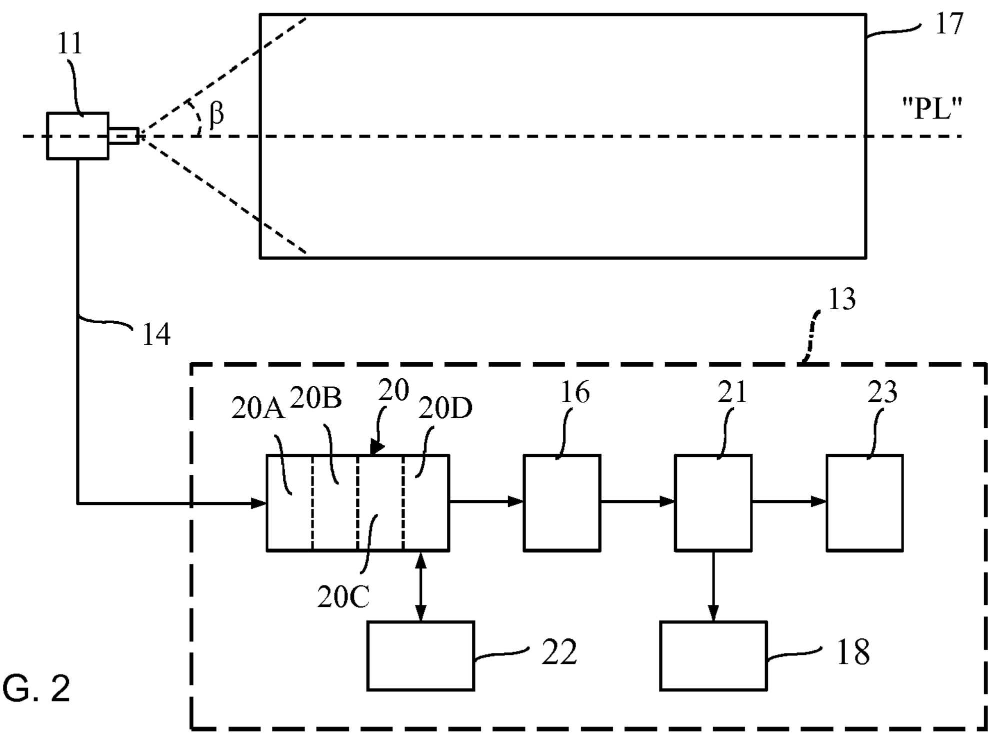
11
17
β
"PL"
14
13
20A
20B
20
20D
16
21
23
20C
22
18

FIG. 2

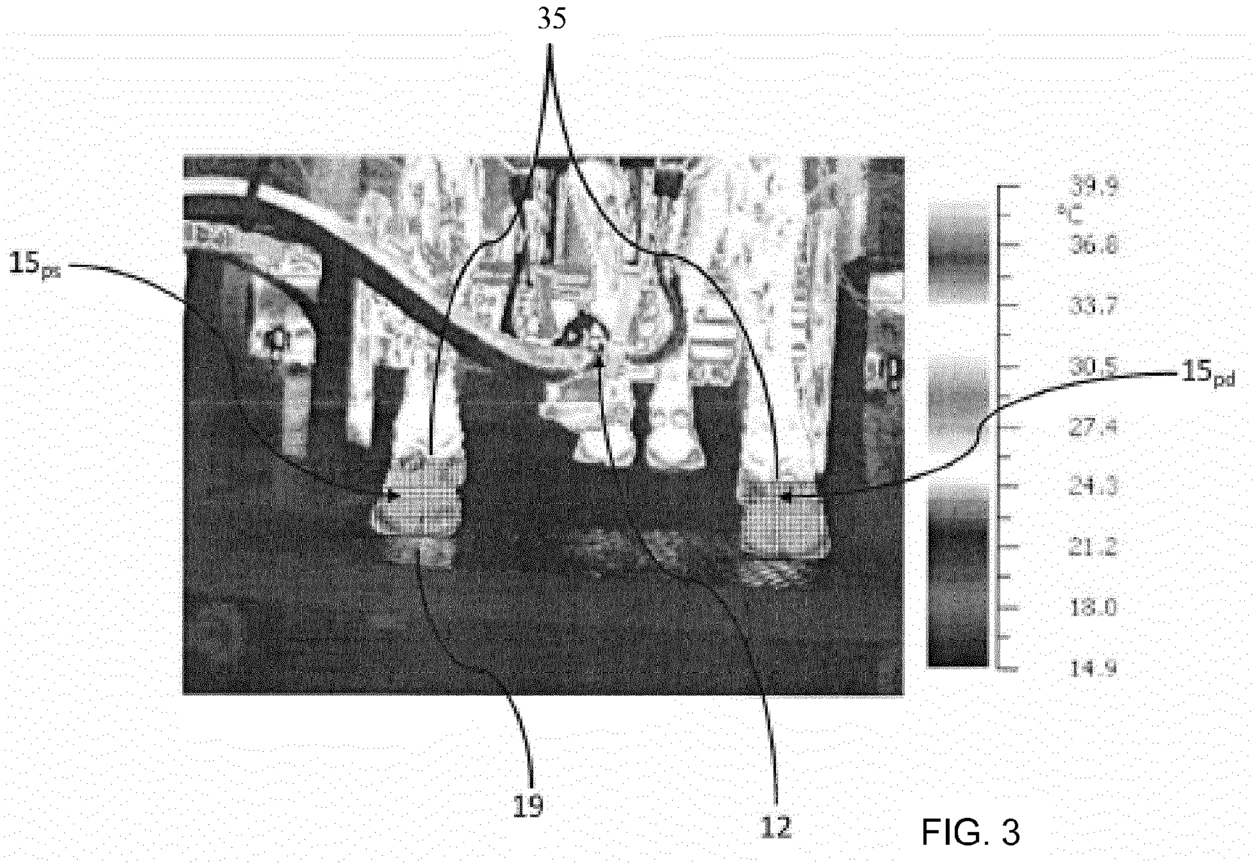
35
15ps
15pd
°C
39.9
36.8
33.7
30.5
27.4
24.3
21.2
18.0
14.9
19
12

FIG. 3

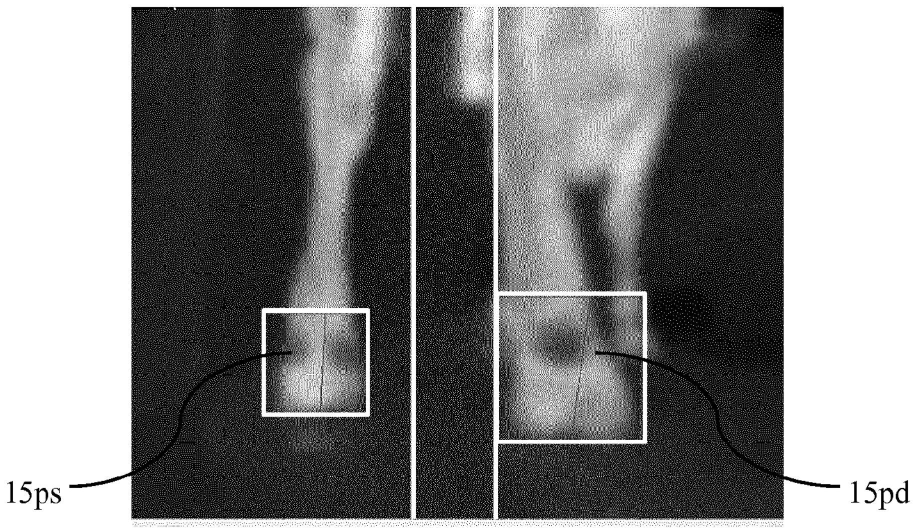
15ps
15pd

FIG. 4

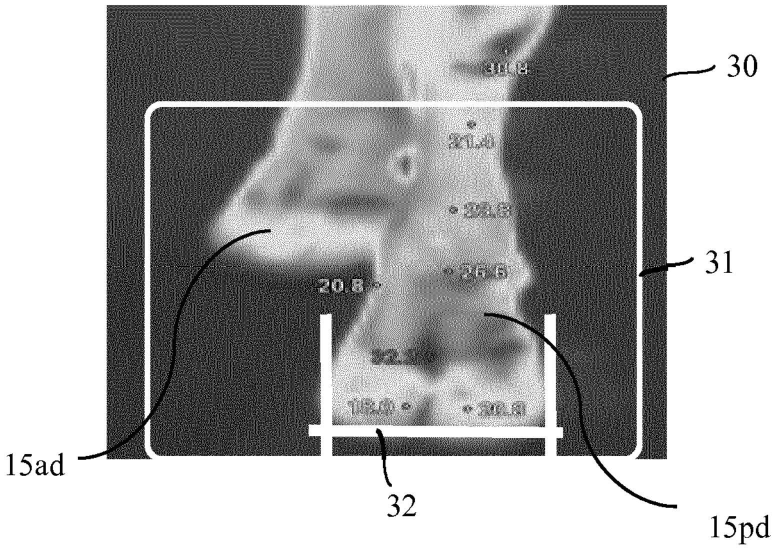


FIG. 5

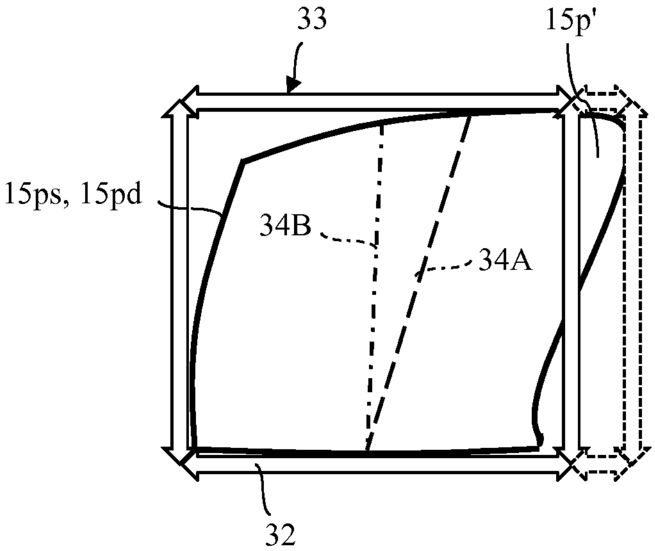


FIG. 8

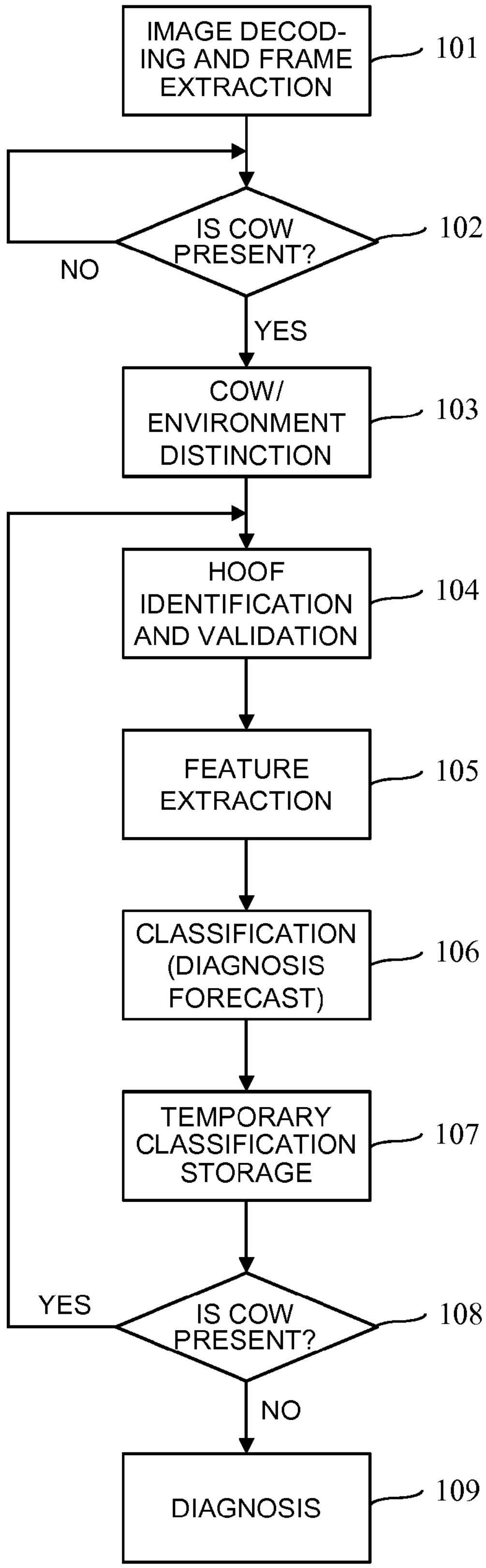
IMAGE DECODING AND FRAME EXTRACTION
101
IS COW PRESENT?
102
NO
YES
COW/ ENVIRONMENT DISTINCTION
103
HOOF IDENTIFICATION AND VALIDATION
104
FEATURE EXTRACTION
105
CLASSIFICATION (DIAGNOSIS FORECAST)
106
TEMPORARY CLASSIFICATION STORAGE
107
IS COW PRESENT?
108
YES
NO
DIAGNOSIS
109

FIG. 6

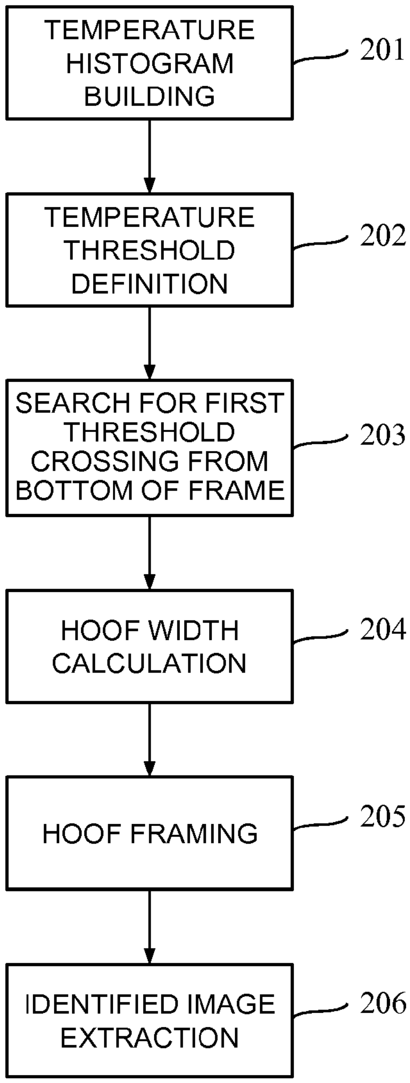
TEMPERATURE HISTOGRAM BUILDING
201
TEMPERATURE THRESHOLD DEFINITION
202
SEARCH FOR FIRST THRESHOLD CROSSING FROM BOTTOM OF FRAME
203
HOOF WIDTH CALCULATION
204
HOOF FRAMING
205
IDENTIFIED IMAGE EXTRACTION
206

FIG. 7

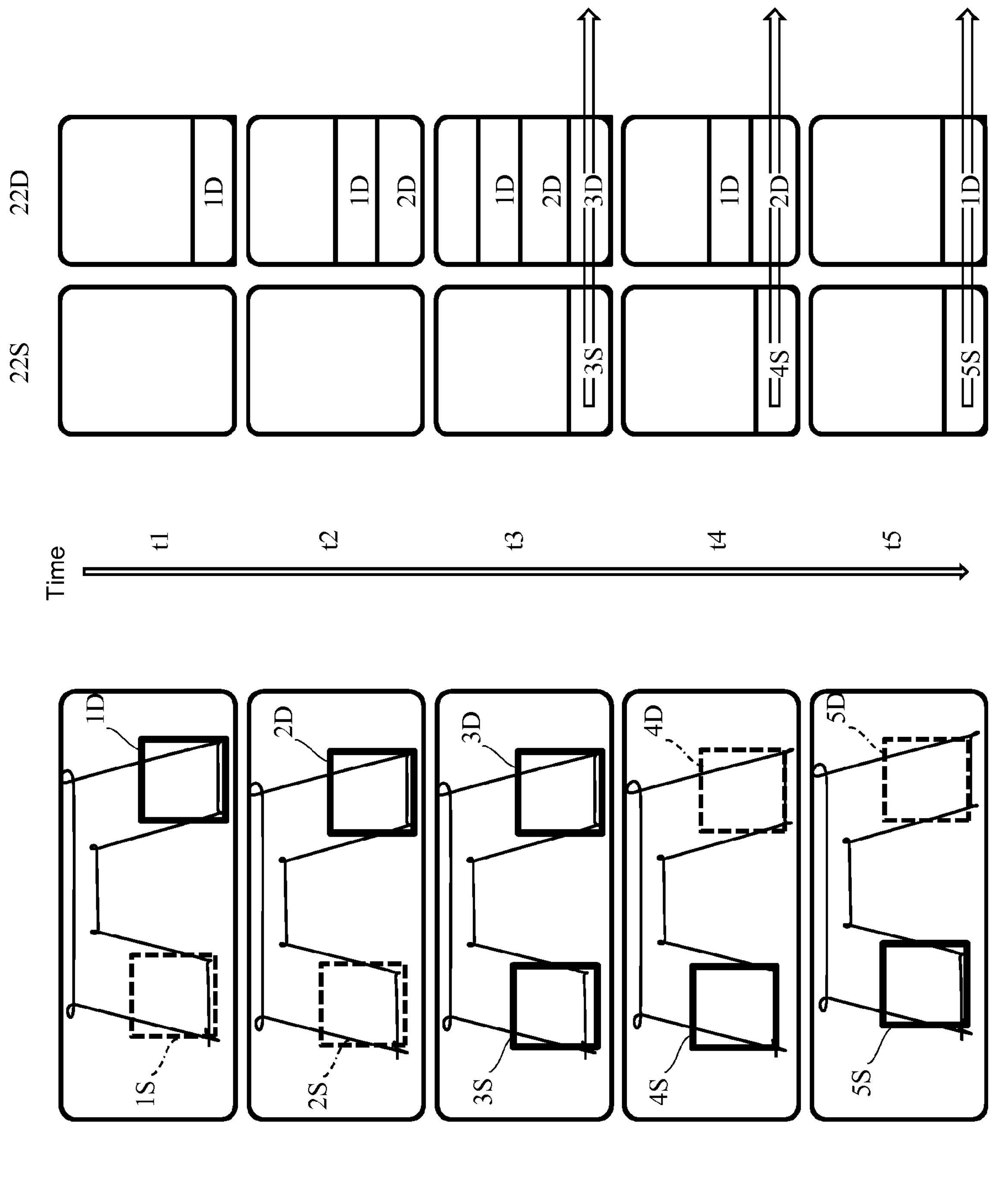
22S
22D
1D
1D
2D
1D
2D
3D
3S
1D
2D
4S
1D
5S
Time
t1
t2
t3
t4
t5
1D
1S
2D
2S
3D
3S
4D
4S
5D
5S

FIG. 9

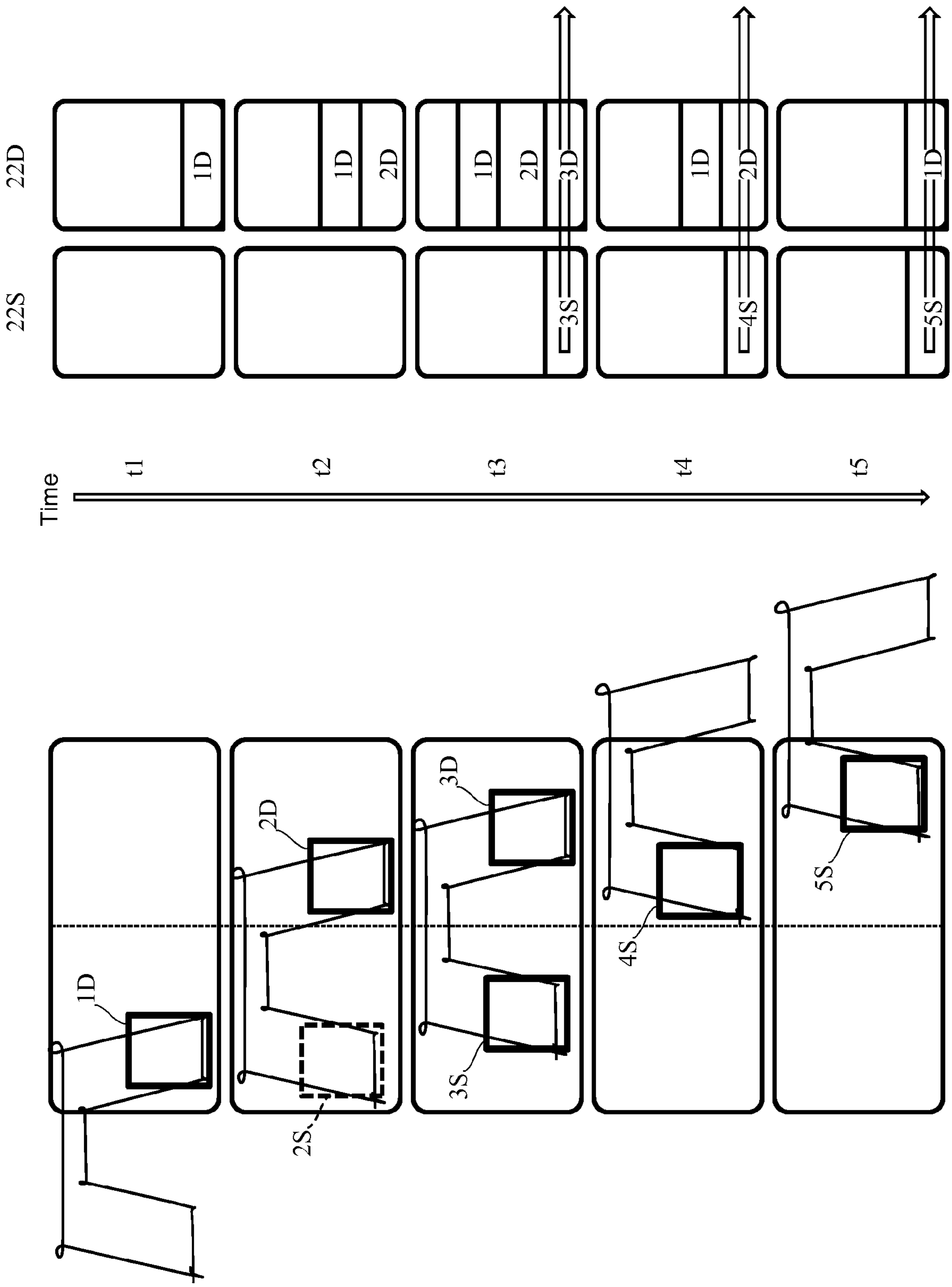
22S
22D
1D
1D
2D
1D
2D
3D
3S
1D
2D
4S
1D
5S
Time
t1
t2
t3
t4
t5
1D
2D
2S
3D
3S
4S
5S

FIG. 10

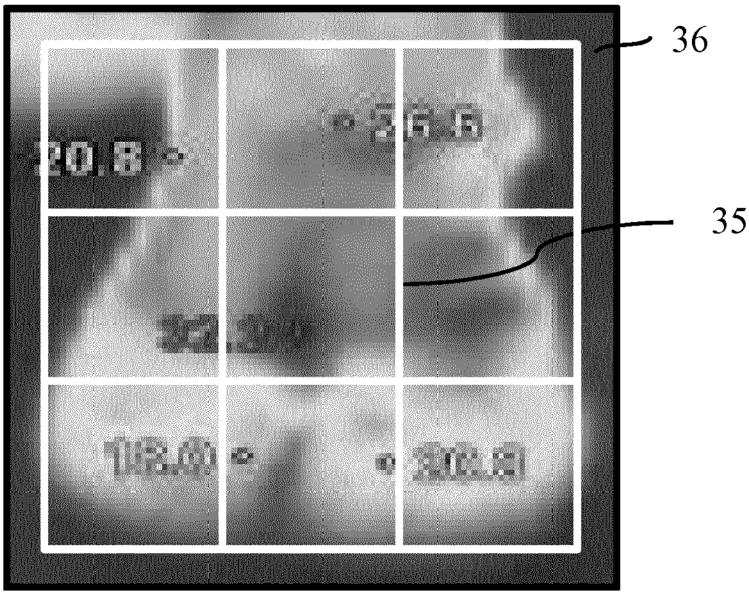


FIG. 11

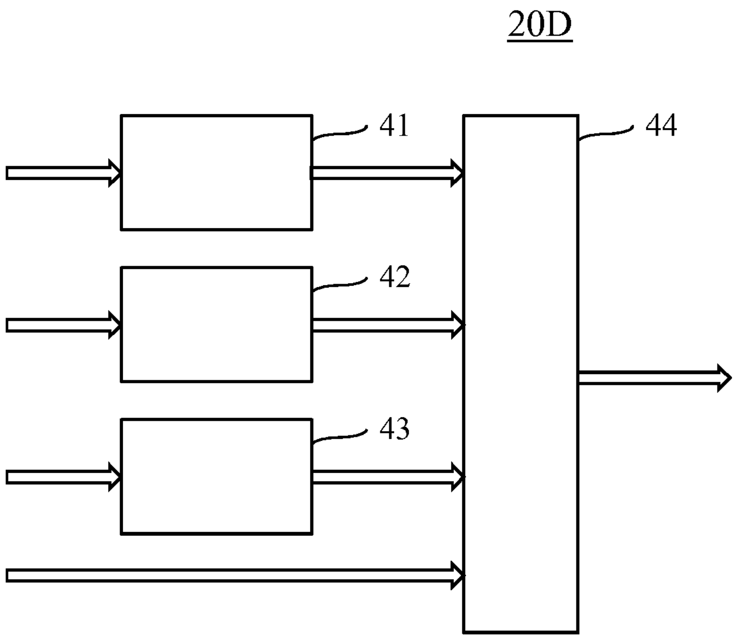


FIG. 12

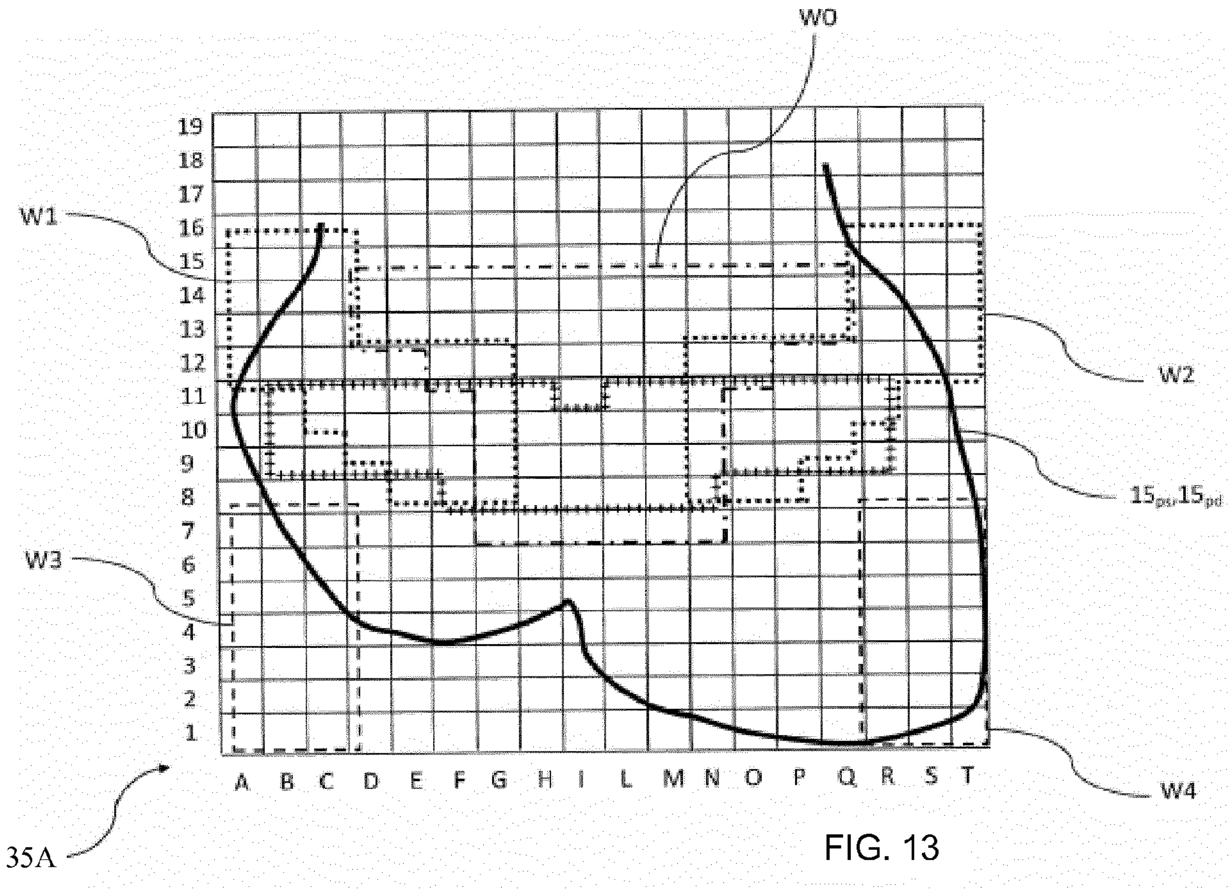


FIG. 13

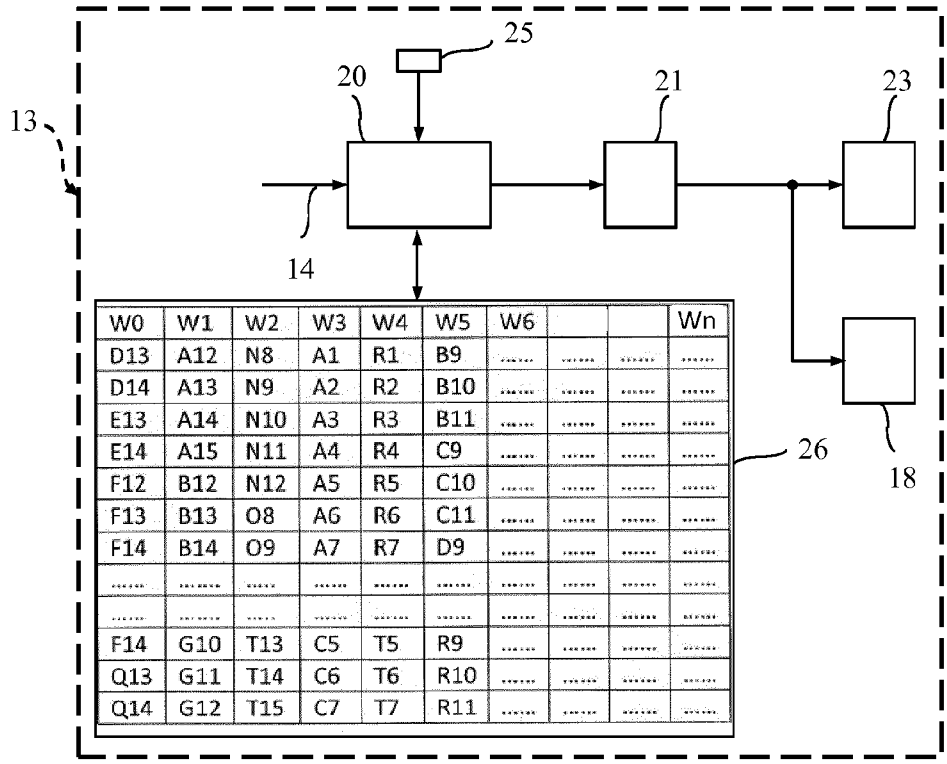


| W0 | W1 | W2 | W3 | W4 | W5 | W6 | | | Wn |
|---|---|---|---|---|---|---|---|---|---|
| D13 | A12 | N8 | A1 | R1 | B9 | ...... | ...... | ...... | ...... |
| D14 | A13 | N9 | A2 | R2 | B10 | ...... | ...... | ...... | ...... |
| E13 | A14 | N10 | A3 | R3 | B11 | ...... | ...... | ...... | ...... |
| E14 | A15 | N11 | A4 | R4 | C9 | ...... | ...... | ...... | ...... |
| F12 | B12 | N12 | A5 | R5 | C10 | ...... | ...... | ...... | ...... |
| F13 | B13 | O8 | A6 | R6 | C11 | ...... | ...... | ...... | ...... |
| F14 | B14 | O9 | A7 | R7 | D9 | ...... | ...... | ...... | ...... |
| ...... | ...... | ...... | ...... | ...... | ...... | ...... | ...... | ...... | ...... |
| ...... | ...... | ...... | ...... | ...... | ...... | ...... | ...... | ...... | ...... |
| F14 | G10 | T13 | C5 | T5 | R9 | ...... | ...... | ...... | ...... |
| Q13 | G11 | T14 | C6 | T6 | R10 | ...... | ...... | ...... | ...... |
| Q14 | G12 | T15 | C7 | T7 | R11 | ...... | ...... | ...... | ...... |

FIG. 14

Europäisches Patentamt
European Patent Office
Office européen des brevets

# EUROPEAN SEARCH REPORT

Application Number
EP 16 17 8313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | M. ALSAAOD ET AL: "Detection of hoof lesions using digital infrared thermography in dairy cows", JOURNAL OF DAIRY SCIENCE., vol. 95, no. 2, 1 February 2012 (2012-02-01), pages 735-742, XP055246317, US ISSN: 0022-0302, DOI: 10.3168/jds.2011-4762 * /* Material and Methods p. 736-738 */ * ----- | 1-4,7, 9-15 | INV. A61B5/01 |
| A | J.E. STOKES ET AL: "An investigation into the use of infrared thermography (IRT) as a rapid diagnostic tool for foot lesions in dairy cattle", VETERINARY JOURNAL, vol. 193, no. 3, 1 September 2012 (2012-09-01), pages 674-678, XP055246307, GB ISSN: 1090-0233, DOI: 10.1016/j.tvjl.2012.06.052 * /* Materials and Methods p. 675-677 */ * ----- | 1-15 | TECHNICAL FIELDS SEARCHED (IPC) A61B |
| A | S. WOOD ET AL: "Infrared thermometry for lesion monitoring in cattle lameness", VETERINARY RECORD., vol. 176, no. 12, 2 December 2014 (2014-12-02), pages 308-308, XP055331786, GB ISSN: 0042-4900, DOI: 10.1136/vr.102571 * /* Material and methods p. 2-3 */ * ----- -/-- | 1-15 | |

The present search report has been drawn up for all claims

2

| Place of search | Date of completion of the search | Examiner |
|---|---|---|
| Berlin | 11 January 2017 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 16 17 8313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Ina Pampariene ET AL: "DETERMINATION OF COWS' HOOVES HEALTH APPLYING THERMOGRAPHY", VETERINARIJA IR ZOOTECHNIKA, 1 January 2014 (2014-01-01), pages 60-68, XP055331795, Retrieved from the Internet: URL:http://vetzoo.lsmuni.lt/data/vols/2014/68/pdf/pampariene.pdf [retrieved on 2017-01-03] * /* Material and methods p.61-62 */ * ----- | 1-15 | |

TECHNICAL FIELDS SEARCHED (IPC)

The present search report has been drawn up for all claims

2

| Place of search | Date of completion of the search | Examiner |
|---|---|---|
| Berlin | 11 January 2017 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2004158156 A1

### Non-patent literature cited in the description

- **T.A. TURNER ; R.C. PUROHIT ; J.F. FESSLER.** Thermography: A review in equine medicine. *Comp. Cont. Ed.,* 1986, vol. 8, 855
- **WEIL, M. ; LITZKE, L.F. ; FRITSCH, R.** Diagnostic validity of thermography in equine lameness. *Tierarztliche Praxis Ausgabe G: Grosstiere - Nutztiere,* 1998, vol. 26 (6), 346-354
- **SIMONE WESTERMANN et al.** Effects of infrared camera angle and distance on measurement and reproducibility of thermographically determined temperatures of the distolateral aspects of the forelimbs in horses. *Journal of the American Veterinary Medical Association,* 01 February 2013, vol. 242 (3
- **A.L. EDDY ; L.M. VAN HOOGMOED ; J. R. SNYDER.** The Role of Thermography in the Management of Equine Lameness. *The Veterinary Journal,* 2001, vol. 162, 172-181
- **M. ALSAOOD et al.** Detection of hoof lesions using digital infrared thermography in dairy cows. *Journal of Dairy Science,* 2012, vol. 95 (2), 735-742
- **M. ALSAOOD et al.** A field trial of infrared tomography as a non-invasive diagnostic tool for early detection of digital dermatitis in dairy cows. *The Veterinary Journal,* 2014, vol. 199, 281-285